Europäisches Patentamt

European Patent Office

Office européen des brevets

(11) Veröffentlichungsnummer: **0 496 991 A1**

## EUROPÄISCHE PATENTANMELDUNG

(21) Anmeldenummer: **91121592.9**

(22) Anmeldetag: **17.12.91**

(51) Int. Cl.5: **A61M 39/00, A61M 39/02**

(30) Priorität: **31.01.91 DE 4102775**

(43) Veröffentlichungstag der Anmeldung:
**05.08.92 Patentblatt 92/32**

(84) Benannte Vertragsstaaten:
**AT BE CH DE DK ES FR GB GR IT LI LU NL SE**

(71) Anmelder: **B. Braun Melsungen AG
Carl-Braun Strasse
W-3508 Melsungen(DE)**

(72) Erfinder: **Knauf, Bernhard
Buchenhain 25
W-3501 Koerle(DE)**

(74) Vertreter: **Selting, Günther, Dipl.-Ing. et al
Patentanwälte von Kreisler, Selting, Werner
Deichmannhaus am Hauptbahnhof
W-5000 Köln 1(DE)**

(54) **Anschlusseinheit für einen Katheterschlauch.**

(57) Die Anschlußeinheit für einen Katheterschlauch (10) weist einen Anschlußstutzen (20) mit Innengewinde (22) und ein in den Anschlußstutzen (20) einschraubbares Druckstück mit einem Axialkanal für den Katheterschlauch (10) auf. Den Katheterschlauch (10) umgibt ein elastomeres Klemmteil (52), das zwischen einer ersten Ringfläche (23) am Anschlußstutzen (20) und einer zweiten Ringfläche am Druckstück axial zusammendrückbar ist, wobei es den Katheterschlauch (10) radial gegen eine starre rohrförmige Stützkanüle (30) preßt, die koaxial verlaufend in dem Anschlußstutzen (20) befestigt ist und den Katheterschlauch (10) von innen abstützt. Die Stützkanüle (30) weist an einem Ende einen Konusstecker (31) auf, der in eine konische Bohrung der Anschlußeinheit (11) passend einsetzbar ist. Der Anwender kann diese Anschlußeinheit mit unterschiedlichen Stützkanüle-/Katheterschlauch-Kombinationen seiner Wahl bestücken.

FIG.4

EP 0 496 991 A1

Die Erfindung bezieht sich auf eine Anschlußeinheit für einen Katheterschlauch, die einen Anschlußstutzen mit Innengewinde und ein in den Anschlußstutzen einschraubbares Druckstück mit einem Axialkanal für den Katheterschlauch aufweist und die ein elastomeres Klemmteil enthält, das den Katheterschlauch umgibt und zwischen einer ersten Ringfläche am Anschlußstutzen und einer zweiten Ringfläche am Druckstück axial zusammendrückbar ist, wobei eine starre rohrförmige Stützkanüle zur inneren Abstützung des Katheterschlauches vorgesehen ist, die von einer Ansatzstelle an der Anschlußeinheit koaxial zum Anschlußstutzen nach außen ragt.

Für gewisse Therapien ist eine ständige Zufuhr von Pharmazeutika in den Körper eines Patienten erforderlich. Zu diesem Zweck wird ein Ende des Katheterschlauches in ein Blutgefäß oder ein Organ eingeführt und das andere Ende des Katheterschlauches wird mit der Anschlußeinheit, z.B. einer Spritze oder eine implantierten bzw. extrakorporalen Kapsel, klemmend verbunden. Die Stützkanüle hat dabei die Aufgabe, ein Zusammenquetschen des Katheterschlauches durch das sich radial einwärts verformende elastomere Klemmteil zu verhindern und die radiale Preßkraft zu verteilen.

Bei der erwähnten bekannten Anschlußeinheit (DE 36 44 916 C1) ist die Stützkanüle über einen Befestigungsabschnitt unlösbar an der Anschlußeinheit befestigt. Der Befestigungsabschnitt ist ein Rohrstück mit eingeschnürtem Mittelbereich, das z.B. von dem Kunststoffmaterial eines Gehäuses der Anschlußeinheit umspritzt ist. Von dem Befestigungsabschnitt geht geradlinig die Stützkanüle aus, die eine Hohlnadel mit zylindrischer Umfangsfläche ist, auf die der Katheterschlauch aufgesteckt ist, dessen Innendurchmesser nur geringfügig größer als der Außendurchmesser der Stützkanüle ist. Dem Anwender steht eine Auswahl von Anschlußeinheiten mit zugehörigen Katheterschläuchen unterschiedlicher Länge zur Verfügung und er kann bei der präoperativen Vorauswahl der Anschlußeinheit, z.B. einer Portkapsel zur Aufnahme eines flüssigen Medikamentes, das durch den Katheterschlauch in einen Patienten eingeleitet werden soll, individuell eine gewünschte Katheterlänge auswählen. Dabei ist jedoch der Katheterdurchmesser festgelegt, da für jede Größe eine zugehörige Portkapsel mit fest eingesetzter Stützkanüle existiert. Bei präoperativer Fehleinschätzung des benötigten Katheterdurchmessers ist der Anwender gezwungen, ein zusätzliches komplettes Set der richtigen Größe zu benutzen, das aus der Portkapsel mit Stützkanüle, dem Katheterschlauch und der Druckstück/Klemmteilkombination besteht. Dies ist kostspielig und es besteht die Gefahr, daß der Einsparung halber ein nicht optimales Set implantiert wird.

Der Erfindung liegt die Aufgabe zugrunde, die Anschlußeinheit nach DE 36 44 916 so zu verbessern, daß sie vom Anwender mit Katheterschläuchen unterschiedlicher Durchmesser ausrüstbar ist.

Diese Aufgabe wird erfindungsgemäß dadurch gelöst, daß an der Ansatzstelle der Anschlußeinheit eine konische Bohrung ausgebildet ist, deren äußeres Ende größeren Durchmessers dem Anschlußstutzen zugewandt ist und daß die Stützkanüle an ihrem dem Anschlußstutzen zugewandten Ende einen Konusstecker aufweist, der in die konische Bohrung passend einsetzbar ist.

Auf diese Weise wird die Anschlußeinheit zu einem Bauteilsystem, das dem Anwender den Zusammenbau eines einzigen Anschlußeinheit-Typs mit unterschiedlichen Stützkanüle-/Katherschlauch-Kombinationen ermöglicht. Der Anwender ist in der Lage, eine für alle Kathetergrößen geeignete universelle Anschlußeinheit intraoperativ mit einem Katheter geeigneten Durchmessers auszurüsten. Das Risiko des Verwerfens und der Neuwahl einer gesamten Anschlußeinheit durch falsche präoperative Vorauswahl entfällt. Die Kosten bleiben auf eine einzige Anschlußeinheit beschränkt. Die Erfindung wirkt sich auch auf die gesamte Produktionslinie aus, weil die Fertigung rationeller erfolgen kann, da nur noch eine einzige Größe der Anschlußeinheit produziert werden muß, die sich über die gleichbleibende Steckverbindung von konischer Bohrung und Konusstecker mit Stützkanülen und Katheterschläuchen unterschiedlicher Durchmesser bestücken läßt. In steriler Verpackung können Katheterschlauch, Stützkanüle und Druckstück mit elastomerem Klemmteil nach Durchmesser und Länge sortiert zu Sets zusammengestellt sein. Der Anwender ist dann in der Lage, sich das optimale Set intraoperativ auszusuchen.

In vorteilhafter Ausgestaltung der Erfindung ist vorgesehen, daß die Länge und der Steigungswinkel der absatzlosen Konusflächen des Konusstekkers und der konischen Bohrung einander angepaßt sind und daß der Steigungswinkel im Bereich von 2° bis 3° liegt. Diese Ausbildung ist vorteilhaft, weil sie eine gute Haftung des Konussteckers in der konischen Bohrung gewährleistet und sicherstellt, daß die Stützkanüle zentriert in dem Anschlußstutzen verläuft, so daß der auf die Stützkanüle aufgeschobene Katheterschlauch ringsum gleichmäßig von dem zusammengedrückten elastomeren Klemmteil gepreßt und gehalten wird. Der Katheterschlauch erfährt auf diese Weise keine örtlich verstärkten Pressungen und seine Haltbarkeit bleibt unbeeinträchtigt. Außerdem sorgt die Gleichmäßigkeit der Einspannung des Katheterschlauches zwischen Stützkanüle und elastomerem Klemmstück für dauernde gute Abdichtung der Anschlußeinheit.

Bevorzugt ist ein zylindrischer Schaft der Stützkanüle mit dem Konusstecker einstückig ausgebildet. Er besteht vorzugsweise aus Stahl. Während die Abmessungen des Konussteckers bei jeder Stützkanüle gleich sind, damit er in die unveränderliche konische Bohrung aller Anschlußeinheiten paßt, hat der Schaft der Stützkanüle, auf den der Katheterschlauch aufgeschoben ist, einen diesem angepaßten Außendurchmesser. Der Konusstecker schließt sich über eine vorzugsweise rechtwinklig zur Längsachse gerichtete radiale Ringschulter an den Schaft der Stützkanüle an. Die Richtung der Ringschulter hängt davon ab, ob der Durchmesser des Schaftes größer oder kleiner als der Durchmesser des breiten Endes des Konussteckers ist. Bei kleinerem Durchmesser des Schaftes ist die Ringschulter dem Schaft zugewandt und sie bildet eine Stufe, gegen die die Stirnfläche des aufgeschobenen Katheterschlauches anstößt. Auch die Stirnfläche des den Katheterschlauch umgebenden elastomeren Klemmteiles kann mit einer inneren Radialzone gegen die Ringschulter zur Anlage kommen. Eine solche Dimensionierung ist günstig, weil sie die Verpackung von Druckstück, Klemmteil, Katheterschlauch und Stützkanüle als montierte zusammenbleibende Einheit ermöglicht, die sich von dem Anwender als Ganzes erfassen und mit der Anschlußeinheit durch Zusammenstecken und Festschrauben des Druckstückes auf einfache Weise verbinden läßt.

Der Schaft der Stützkanüle ist länger als die addierte Länge von Druckstück und Klemmteil, so daß im montierten Zustand ein Abschnitt des Schaftes über das Druckstückende vorsteht. Dies hat den Vorteil, daß der Katheterschlauch noch ein Stück jenseits des Druckstückes von innen abgestützt ist und an der Austrittsstelle aus dem Axialkanal des Druckstückes nicht abknicken kann.

Um zu vermeiden, daß der dünne empfindliche Katheterschlauch mit der harten Wand des Axialkanals des Druckstückes in Berührung kommt und dabei möglicherweise Schaden nimmt, ist erfindungsgemäß der Axialkanal des Druckstückes mit einem Schlauchkörper ausgekleidet, der über die Ringfläche des Druckstückes mit einem Verdickungsabschnitt übersteht, der das Klemmteil bildet. Bei axialer Zusammendrückung des Verdickungsabschnittes verformt er sich radial einwärts und klemmt den Katheterschlauch auf der Stützkanüle schonend fest. Gleichzeitig ergibt sich eine gewisse Materialverlagerung von dem Verdickungsabschnitt in den auskleidenden Teil des Schlauchkörpers hinein, so daß auch dieser radial schwillt und sich abdichtend gegen die Katheterschlauchaußenfläche anlegt. Der Schlauchkörper ist vorzugsweise in das Druckstück eingespritzt. Seiner zuverlässigen Fixierung in dem Druckstück dient, daß der Schlauchkörper an seinem dem Verdickungsabschnitt abgewandten Ende mit einer äußeren Umfangsmanschette versehen ist, die in eine angepaßte Aussparung des Axialkanals des Druckstückes eingreift. Das Lumen des Schlauchkörpers der Auskleidung erweitert sich gegen die Umfangsmanschette, damit das Entformen erleichtert wird. Außerdem wird durch diese Durchgangserweiterung das Hineinstecken des Katheterschlauches in den Axialkanal des Druckstückes vereinfacht.

In der Zeichnung sind Ausführungsbeispiele der Erfindung schematisch dargestellt. Es zeigt:

Fig. 1 einen Schnitt durch eine als implantierbare Portkapsel ausgebildete Anschlußeinheit für einen Katheterschlauch,

Fig. 2 einen Längsschnitt durch eine Stützkanüle mit Konusstecker,

Fig. 3 einen Längsschnitt durch ein mit einer Stützkanüle nach Fig. 2, einem Katheterschlauch und einem Klemmteil ausgerüstetes Druckstück und

Fig. 4 einen Schnitt durch eine mit der Anordnung nach Fig. 3 ausgerüstete Anschlußeinheit.

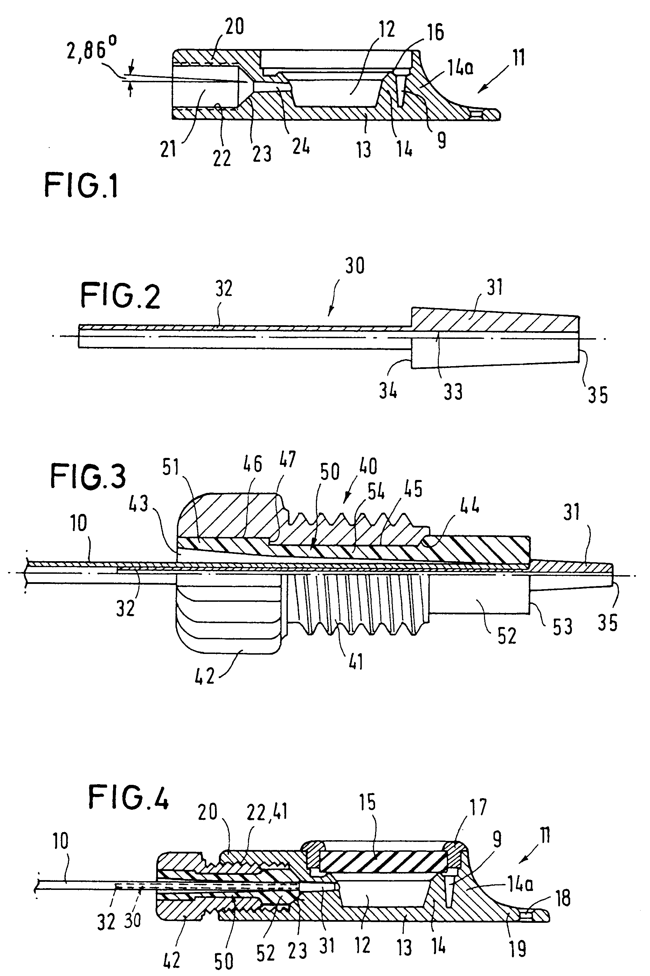

Eine Anschlußeinheit für einen Katheterschlauch 10 in Form eines flexiblen Schlauches weist eine in Draufsicht kreisförmige Kapsel 11 auf, in deren Zentrum ein kreisförmiger Hohlraum 12 ausgebildet ist. Der Hohlraum 12 dient zur Aufnahme eines flüssigen Medikamentes, das durch den Katheterschlauch 10 in einen Körperhohlraum, z.B. ein Blutgefäß, eines Patienten eingeleitet werden soll. Der Hohlraum 12 ist umschlossen von einer Bodenwand 13, einer Umfangswand 14 und einer perforierbaren Oberwand 15 (Fig. 4) aus gummielastischem Material, die sich nach Durchstechen mit einer Hohlnadel zur Befüllung des Hohlraumes 12 selbsttätig wieder verschließt. Die Oberwand 15 liegt mit ihrer ebenen Unterfläche auf einem scharfen ringförmigen Rand 16 am freien oberen Ende der Umfangswand 14 auf und wird von einem gegen die Außenfläche ihres Randes andrückenden Befestigungsring 17 gegen den Rand 16 abgedichtet angepreßt und gehalten. Der Befestigungsring 17 ist in eine Ringvertiefung eingepaßt und auf die Außenwand 14a dicht aufgesetzt und mit dieser verschweißt oder verklebt. Zwischen der Umfangswand 14 und der Außenwand 14a verläuft ein konischer kreisförmiger Ringraum 9. Die Außenwand 14a umgibt die Umfangswand 14 des Hohlraumes 12 konzentrisch und ihre Außenfläche geht mit einer konkaven Wölbung in einen radial abstehenden ringförmigen Flansch 19 über, der die Bodenwand 13 umgibt und sich in der gleichen Ebene wie diese befindet. Im Randbereich des Flansches 19 sind Löcher 18 zum Fixieren der Kapsel 11 an der Faszie ausgebildet. Die Unterseite der Bodenwand 13 und des Flansches 19 ist glattflächig und eben.

An den Flansch 19 und die Außenwand 14a ist ein radialer Anschlußstutzen 20 einstückig angeformt. Der Anschlußstutzen 20 erhebt sich von dem Flansch 19 nach oben und endet an der Nahtstelle zwischen der Außenwand 14a und dem Befestigungsring 17. Der Anschlußstutzen 20 steht nicht über den Flansch 19 hinaus seitlich vor, so daß er die radialen Abmessungen der Kapsel 11 nicht vergrößert. Er enthält einen kreiszyllndrischen Raum 21, dessen Umfangswand über ihre ganze Länge mit einem Innengewinde 22 versehen ist. Der Raum 21 endet innen in einer kegelstumpfförmigen Fläche 23, von deren axialer Mitte eine konische Bohrung 24 ausgeht. Das äußere Ende größeren Durchmessers der konischen Bohrung 24 ist dem Raum 21 zugewandt und ihr inneres Ende kleineren Durchmessers mündet in den Hohlraum 12 der Kapsel 11. Die Längsachse der konischen Bohrung 24 verläuft parallel zur Bodenwand 13 und koaxial mit der Mittelachse des Raumes 21. Die konische Bohrung 24 befindet sich in einem Radialsteg, der den kreisförmigen Ringraum 9 zwischen der Umfangswand 14 und der Außenwand 14a überbrückt. Der größere Durchmesser der konischen Bohrung 24 beträgt bevorzugt 1,4 mm und der Steigungswinkel der Wandung der konischen Bohrung 24 ist günstigerweise 2,86°.

Die konische Bohrung 24 dient als negativer Sitz für einen Konusstecker 31 einer starren rohrförmigen Stützkanüle 30, die sich koaxial durch den Raum 21 und über diesen hinaus erstreckt. Die Stützkanüle 30 ist vorzugsweise aus Stahl hergestellt und mit dem Konusstecker 31 ist einstükkig ein gerader zylindrischer Schaft 32 verbunden, der als Stützabschnitt für den Katheterschlauch 10 dient. Die Außenfläche des Schaftes 32 und des Konussteckers 31 ist glatt. Der Steigungswinkel der Außenfläche des Konussteckers 31 entspricht dem Steigungswinkel der konischen Bohrung 24, d.h. er ist ebenfalls 2,86°. Der Durchmesser des äußeren Endes größeren Durchmessers des Konussteckers 31 beträgt bevorzugt 1,5 mm, während das innere Ende kleineren Durchmessers einen Durchmesser von 1,1 mm hat. Auf diese Weise ist gewährleistet, daß der Konusstecker 31 festhaftend in der konischen Bohrung 24 steckt und den Schaft 32 der Stützkanüle 30 koaxial und unabgestützt nach außen ragend festhält. Die Stützkanüle 30 enthält einen durchgehenden geraden Längskanal 33, der den Hohlraum 12 mit der Umgebung verbindet.

Auf den Schaft 32 der Stützkanüle 30 ist das in dem Anschlußstutzen festzulegende Ende des Katheterschlauches 10 aufgeschoben, dessen Innendurchmesser dem Außendurchmesser des Schaftes 32 der Stützkanüle 30 so angepaßt ist, daß der Katheterschlauch 10 den Schaft 32 spannungsfrei, jedoch von innen abgestützt, umgibt. Das innere Ende des Katheterschlauches 10 stößt gegen die Ringschulter 34 der Stützkanüle 30 an.

Das äußere Ende größeren Durchmessers des Konussteckers 31 schließt sich über eine Ringschulter 34 an den Schaft 32 an. Die Ringschulter 34 verläuft rechtwinklig zu der Außenfläche des Schaftes 32 radial nach außen und parallel zur inneren Stirnfläche 35 des Konussteckers 31. Die axiale Länge des Konussteckers 31 ist so bemessen, daß die Stirnfläche 35 des Konussteckers 31 nicht in den Hohlraum 12 vorsteht, sondern mit dessen Innenwandung etwa abschließt.

Auf den von innen abgestützte Katheterschlauch 10 ist ein Druckstück 40 aufgeschoben, das aus hartem Kunststoff besteht und einen Gewindeschaft 41 mit Außengewinde aufweist, an dessen rückwärtigem Ende ein Drehknopf 42 mit Greifrippen ausgebildet ist. Das Druckstück 40 ist von einem an beiden Enden offenen Axialkanal 43 durchsetzt, dessen Wandung in drei axial aufeinanderfolgende Abschnitte unterteilt ist, und zwar eine trichterförmige vordere Ringfläche 44, einen kreiszylindrischen geraden Teil 45 und eine kreiszylindrische Aussparung 46, deren Durchmesser größer ist als derjenige des Teiles 45 und die über eine Ringstufe 47 an den Teil 45 anschließt. Der Axialkanal 43 des Druckstückes 40 ist durch einen eingesetzten Schlauchkörper 50 aus Elastomermaterial verengt. Der Schlauchkörper 50 kann in das Druckstück 40 eingespritzt sein. Mit einer äußeren Umfangsmanschette 51 greift er in die Aussparung 46 ein und auch der Ringfläche 44 ist er angepaßt. Von dieser geht der Schlauchkörper in einen Verdickungsabschnitt über, der ein Klemmteil 52 bildet. Das Klemmteil 52 hat in Bezug auf den restlichen Teil der Länge des Schlauchkörpers 50 beträchtliche radiale Dicke. Es ist kreiszylindrisch gestaltet und endet in einer ebenen Stirnfläche 53.

Der Durchlaß in dem Schlauchkörper 50 bildet den an beiden Enden offenen, geraden Axialkanal 43 des Druckstückes 40. Er erweitert sich von der Lochöffnung an der Stirnfläche 53 gegen das Ende des Drehknopfes 42 konisch. Der Durchmesser des Axialkanales 43 entspricht an der Stirnfläche 53 dem Außendurchmesser des Katheterschlauches 10, so daß dieser an dieser Stelle eng von dem Klemmteil 52 umschlossen ist. Eine kurze innere Radialzone des Klemmteils 52 an der Stirnfläche 53 stößt gegen die Ringschulter 34 des Konussteckers 31 an und bildet einen Anschlag, der ein Herausrutschen der Stützkanüle 30 und des Katheterschlauches 10 in Richtung des Drehknopfes 42 verhindert, wenn diese von dem Anwender beim Ansetzen an die Kapsel 11 gehandhabt werden.

Der Raum 21 des Anschlußstutzens 20 wird in seinem hohlraumnahen inneren Bereich von dem Klemmteil 52 ausgefüllt und dieser Bereich bildet einen Klemmraum, dessen Umfangswand von einem Teil des Innengewindes 22, der kegelstumpfförmigen Fläche 23 und der Ringfläche 44 gebildet ist. Bei axialer Zusammenpressung des Klemmteiles 52 durch Drehung des Druckstückes 40 wird durch radiale Deformation eine vielseitige Abdichtung erreicht, d.h. es kann Flüssigkeit weder an dem Katheterschlauch 10 noch an der Wand des Hohlraumes 21 entlang aus der Kapsel 11 austreten. Da die radiale Deformation des Klemmteiles 52 sich durch Materialverdrängung in Richtung des langgestreckten Teiles 54 des Schlauchkörpers 50 fortsetzt und durch Torsion des Schlauchkörpers 50 infolge der Drehung des Druckstückes 40 verstärkt wird, ergibt sich eine lange Strecke abdichtender Anlegung des Schlauchkörpers 50 gegen die Außenfläche des Katheterschlauches 10 und die Abdichtwirkung wird gesteigert. Diese Verteilung der radialen Anpressung ist außerdem günstig, weil sie schädliche örtliche Einquetschungen des Katheterschlauches 10 zwischen dem Schaft 32 der Stützkanüle 30 und dem Klemmteil 52 verhindert.

Die in Fig. 3 gezeigte Anordnung steht dem Anwender als Set zur Verfügung, dessen Katheterschlauch 10 eine dem Anwender geeignet erscheinende Länge und einen gewünschten Durchmesser hat und es wird vom Anwender eine Kapsel 11 standardisierter Größe mit dem Katheterschlauch 10 dadurch verbunden, daß das Druckstück 40 in den Anschlußstutzen 20 eingeschraubt wird, wobei der Konusstecker 31 in die konische Bohrung 24 eindringt und die Stirnfläche 53 des Klemmteiles 52 gegen die kegelstumpfförmige Fläche 23 des Raumes 21 des Anschlußstutzens 20 zur Anlage kommt, wobei es zwischen der Fläche 23 und der Ringfläche 44 axial zusammengedrückt wird. Diese axiale Zusammendrückung hat die radiale Deformation des Klemmteiles 52 zur Folge, die sich in gleichmäßiger Anpressung gegen den Katheterschlauch in diesem Bereich auswirkt, wodurch die Abdichtung und eine Fixierung des Katheterschlauches in dem Anschlußstutzen 20 erzielt wird. Der Schaft 32 verhindert dabei ein Zusammenquetschen des Katheterlumens. Da der Schaft 32 der Stützkanüle 30 das Druckstück 40 überragt, läßt er sich gut anfassen und dient als Einführhilfe beim Einstecken des Konussteckers 31 in die Bohrung 24.

Die Steckverbindung zwischen Kapsel 11 und Stützkanüle 30 erlaubt dem Anwender intraoperativ die Wahl eines Katheters geeigneten Durchmessers und geeigneter Länge, so daß mit einer Reihe von Kapseln 11 gleicher Größe und gleichen Typs individuell unterschiedlich starke Katheterschläuche verbunden werden können.

**Patentansprüche**

1. Anschlußeinheit für einen Katheterschlauch (10), die einen Anschlußstutzen (20) mit Innengewinde (22) und ein in den Anschlußstutzen (20) einschraubbares Druckstück (40) mit einem Axialkanal (43) für den Katheterschlauch (10) aufweist und die ein elastomeres Klemmteil (52) enthält, das den Katheterschlauch (10) umgibt und zwischen einer ersten Ringfläche (23) am Anschlußstutzen (20) und einer zeiten Ringfläche (44) am Druckstück (40) axial zusammendrückbar ist, wobei eine starre rohrförmige Stützkanüle (30) zur inneren Abstützung des Katheterschlauches (10) vorgesehen ist, die von einer Ansatzstelle an der Anschlußeinheit koaxial zum Anschlußstutzen (20) nach außen ragt,
**dadurch gekennzeichnet,** daß an der Ansatzstelle der Anschlußeinheit (11) eine konische Bohrung (24) ausgebildet ist, deren äußeres Ende größeren Durchmessers dem Anschlußstutzen (20) zugewandt ist und daß die Stützkanüle (30) an ihrem dem Anschlußstutzen (20) zugewandten Ende einen Konusstecker (31) aufweist, der in die konische Bohrung (24) passend einsetzbar ist.

2. Anschlußeinheit nach Anspruch 1, dadurch gekennzeichnet, daß die Länge und der Steigungswinkel der absatzlosen Konusflächen des Konussteckers (31) und der konischen Bohrung (24) einander angepaßt sind und daß der Steigungswinkel im Bereich von 2° bis 3° liegt.

3. Anschlußeinheit nach Anspruch 1 oder 2, dadurch gekennzeichnet, daß ein zylindrischer Schaft (32) der Stützkanüle (30) mit dem Konusstecker (31) einstückig ausgebildet ist.

4. Anschlußeinheit nach Anspruch 3, dadurch gekennzeichnet, daß der Konusstecker (31) sich über eine radiale Ringschulter (34) an den Schaft (32) der Stützkanüle (30) anschließt.

5. Anschlußeinheit nach Anspruch 3 oder 4, dadurch gekennzeichnet, daß der Schaft (32) der Stützkanüle (30) länger ist als die addierte Länge von Druckstück (40) und Klemmteil (52).

6. Anschlußeinheit nach einem der Ansprüche 1 bis 5, dadurch gekennzeichnet, daß der Axialkanal (43) des Druckstückes (40) mit einem Schlauchkörper (50) ausgekleidet ist, der über die Ringfläche (44) des Druckstückes axial mit einem Verdickungsabschnitt übersteht, der das Klemmteil (52) bildet.

7. Anschlußeinheit nach Anspruch 6, dadurch gekennzeichnet, daß der Schlauchkörper (50) an seinem dem Verdickungsabschnitt abgewandten Ende mit einer äußeren Umfangsmanschette (51) versehen ist, die in eine angepaßte Aussparung (46) des Axialkanales (43) des Druckstückes (40) eingreift, und daß der Schlauchkörper (50) in das Druckstück (40) eingespritzt ist.

FIG.1

FIG.2

FIG.3

FIG.4

Europäisches
Patentamt

# EUROPÄISCHER RECHERCHENBERICHT

Nummer der Anmeldung

EP 91 12 1592

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 343 910 (SHILEY INFUSAID INC.)<br>* Spalte 6, Zeile 20 - Zeile 43; Anspruch 1; Abbildungen 1,5 *<br>--- | 1,3,4 | A61M39/00<br>A61M39/02 |
| D,A | DE-C-3 644 916 (B.BRAUN MELSUNGEN AG)<br>* Zusammenfassung; Abbildung 1 *<br>--- | 1 | |
| A | US-A-4 187 848 (TAYLOR)<br>* Spalte 3, Absatz 2; Anspruch 1; Abbildungen 3,5 *<br>----- | 1,6,7 | |

| | | | RECHERCHIERTE SACHGEBIETE (Int. Cl.5) |
|---|---|---|---|
| | | | A61M |

Der vorliegende Recherchenbericht wurde für alle Patentansprüche erstellt

| Recherchenort | Abschlußdatum der Recherche | Prüfer |
|---|---|---|
| BERLIN | 16 APRIL 1992 | ROLAND A. |

EPO FORM 1503 03.82 (P0403)